# EUROPEAN PATENT SPECIFICATION

(11) **EP 3 076 997 B1**
(45) Date of publication and mention of the grant of the patent: **29.08.2018**
(21) Application number: 14808935.2
(22) Date of filing: 02.12.2014
(51) Int. Cl.: A61K 39/02, A61K 39/12, A61P 31/14, A61P 31/04

(54) **SWINE VACCINE AGAINST PRRS AND LAWSONIA INTRACELLULARIS**
SCHWEINEIMPFSTOFF GEGEN PPRS UND LAWSONIA INTRACELLULARIS
VACCIN PORCIN CONTRE PRRS ET LAWSONIA INTRACELLULARIS

(30) Priority: 03.12.2013 EP 13195529
(43) Date of publication of application: 12.10.2016
(73) Proprietor: Intervet International B.V., 5831 AN Boxmeer (NL)
(72) Inventor: DREXLER, Christa, NL-5831 AN Boxmeer (NL); JACOBS, Antonius Arnoldus Christiaan, NL-5831 AN Boxmeer (NL)
(74) Representative: Intervet International B.V.
(86) International application number: PCT/EP2014/076243
(87) International publication number: WO 2015/082465

(56) References cited:
- WO-A1-2007/116032
- WO-A1-2009/127684
- WO-A2-2008/073464
- US-A1- 2013 266 603

## Description

### GENERAL FIELD OF THE INVENTION

The invention in general pertains to the field of swine health. Swine are prone to many pathogenic micro-organisms. Control of infection is commonly done by stable and feed management, treatment with pharmaceuticals such as anti-viral drugs and antibiotics, or prophylactic treatment using vaccines.

### OBJECT OF THE INVENTION

There is a continuous need for convenient, safe and efficacious means for the management of swine health.

### SUMMARY OF THE INVENTION

In order to meet the object of the invention a new vaccine for the combined protection of swine against infections with various disease causing micro-organisms is devised, the vaccine comprising in combination an adjuvant, a live attenuated PRRS (porcine reproductive and respiratory syndrome) virus and an inactivated *Lawsonia intracellularis* antigen, wherein the inactivated *Lawsonia intracellularis* antigen comprises killed whole cell *Lawsonia intracellularis* bacteria. PRRS virus and *Lawsonia intracellularis* bacteria are both responsible for substantial economic losses due to their negative influence on swine health. Although for both types of micro-organisms drugs as well as vaccines are known and commercially available, there is no combination vaccine available that is suitable for the combined protection against an infection or clinical disease of these pathogens, which vaccine is efficacious, of good quality (e.g. stable, no antigen interference) and at the same time safe for use in young animals. As is commonly known, not aii combinations of antigens contemplated or suggested may lead to a safe and effective combination vaccine. In fact, there is a high level of uncertainty with regard to the stability, safety and efficacy of the combination vaccine, even when the single (monovalent) vaccines are safe and efficacious.

It is noted that the term "combination" does not exclude that the antigens are provided in the combined form of live PRRS virus and killed whole cell *Lawsonia intracellularis* bacteria, only after administration to a subject animal, for example by injecting two separate vaccines at one injection site.

The committee for veterinary medicinal products of the European Agency for the Evaluation of Medicinal Products (EMEA) in its publication "Note for guidance: requirements for combined veterinary products" (EMEA, 2000, CVMP/IWP/52/97-FINAL), stated (page 2/6) that the "development of combined vaccines is not straightforward. Each combination should be developed and studied individually in terms of quality, safety and efficacy". The committee further indicates that the search for a good combination vaccine typically includes the stability and compatibility between the individual components in the combined vaccine, including for example preservatives, excipients and stabilisers, inactivating agents and adjuvants. On page 3, top paragraph, it is stated that "In combined vaccines, the presence of more than one component can often cause an interaction, leading to either a diminished or an increased response to individual components, compared to when the specific component(s) is administered alone.....Such interactions are often immunological in nature, but may also be caused by other factors with less direct effects on the immune system", and also "When an adjuvant is used to augment the immune response to a combined vaccine, special problems may appear."

The U.S. Department of Health and Human Services, Food and Drug Administration, Center for Biologics Evaluation and Research, published in April 1997 a "Guidance for Industry, for the evaluation of combination vaccines for preventable diseases: Production, Testing and Clinical Studies", in which guidance it is stated (page 3, under "Compatibility of Components") that "Experience has shown that combining monovalent vaccines may result in a new combination which is less safe or effective than desirable. Sometimes the components of inactivated vaccines may act adversely on one or more of the active components", indicating that especially an inactivated vaccine may negatively influence the efficacy of a live vaccine, such as for example occurred when combining a live pertussis vaccine and an inactivated poliovirus vaccine that resulted in a vaccine with decreased pertussis potency. It is indicated that any additional components in the vaccine might complicate the safety and potency of the final product when compared to the individual vaccines.

The World Health Organization (WHO) has published an e-learning course called "Vaccine Safety Basics", which in the MODULE 2 contemplates combination vaccines. This module starts with "Licensed combination vaccines undergo extensive testing before approval by national authorities to assure that the products are safe, effective, and of acceptable quality." It is also stated that "With all combinations, manufacturers must therefore evaluate the potency of each antigenic component, the effectiveness of the vaccine components when combined to induce immunity, risk of possible reversion to toxicity, and reaction with other vaccine components." WO2007/116032 discloses a combination vaccine comprising live PRRS virus and *Mycoplasma hyopneumonia* antigen for use in vaccination of *Mycoplasma hyopneumonia* primed pigs. The present invention, next to the vaccine as such, also pertains to the vaccine for use to protect a swine against an infection with PRRS virus and *Lawsonia intracellularis* bacteria, comprising administering the said vaccine.

### DEFINITIONS

*A vaccine* is a constitution that protects against a post vaccination infection with a pathogenic micro-organism, i.e. a constitution that prevents or reduces the infection by the micro-organism, or prevents or reduces a clinical disease that results from the infection, typically by interfering with the micro-organism itself, for example via antibodies, in the vaccinated host. Vaccination thus prevents, or at least diminishes, the level of infection and/or prevents, or at least diminishes, the level of clinical disease resulting from that infection.

*Inactivated antigen* of a wild type bacterium is any substance or compound, other than the live bacterium as such, against which an immunological response is to be elicited, such that the corresponding virulent bacterium or one or more of its virulence factors will be recognized by the host's immune system as a result of this immune response, and are ultimately at least partly neutralized. Typical examples of inactivated antigen of a wild type bacterium are killed whole bacteria (the term "whole" does not exclude that the bacterial cells are, at least partly, ruptured during the killing process, or that an extract or homogenate of the killed whole cell bacteria is actually provided as the antigen in the "killed whole cell bacteria" vaccine), subunits of the bacterium such as surface expressed proteins, and toxins. The latter two may or may not be recombinantly expressed. With regard to *Lawsonia intracellularis,* several types of inactivated antigen are known in the art, and are for example known from WO2009/144088 (killed whole cell bacteria, Examples 1 and 2), WO2005/070958 (sub-units) and WO97/20050 (killed whole cell).

*A live attenuated virus* is a virus that is capable of replicating as such, but is incapable of inducing a full suite of symptoms of the disease that is normally associated with its virulent (often wild-type) pathogenic counterpart. Typically, the live virus does not replicate within a target host, or replicates at a rate which is not significantly detrimental to the host cells, or does not induce a detrimental host response. With regard to PRRS virus, several vaccines are known in the art that comprise a live attenuated virus that is derived from a wild type virus which is attenuated by multiple passaging in an in vitro cultivated host cell line, such as for example Porcilis® PRRS (MSD Animal Health), Ingelvac® PRRS MLV (Boehringer Ingelheim), Amervac-PRRS (Hipra Laboratories), Pyrsvac-183® (Hipra Laboratories) and Fostera® PRRS (Zoetis). In the art other live attenatuated PRRS viruses have been described for example in Veterinary Microbiology, volume 138, issues 1-2, 2 July 2009, Pages 34-40; Veterinary Immunology and Immunopathology, volume 106, issues 3-4, 15 July 2005, Pages 309-319 and in Vaccine, volume 26, issues 29-30, 4 July 2008, Pages 3594-3600.

### EMBODIMENTS OF THE INVENTION

In an embodiment the vaccine is for the protection of a swine against an infection with PRRS virus and *Lawsonia intracellularis* bacteria after a single shot administration. It was advantageously found that a swine is protected against both pathogens even after a single shot administration of the vaccine. This embodiment does not exclude that a follow up vaccination is given, for example 6 to12 months after the first vaccination to renew the level of protection. This follow up vaccination differs from a boost vaccination in a prime-boost vaccination scheme, wherein protection is only obtained after the boost vaccination. In a prime-boost scheme, the two vaccinations are typically 2-3 weeks apart.

The vaccine comprises an adjuvant. It was found that an adjuvant, which is typically used to improve the immune response of inactivated antigens, does not negatively interfere with the live attenuated PRRS virus, nor excessively increase the reactivity to the other antigen, despite the WHO explicitly warns for this type of interference and reactivity in its Vaccine Safety Basics course (see above) on page 1 of the course, last two lines (section "Combination vaccines"). In a further embodiment the adjuvant comprises a mineral oil, such as for example a saturated hydrocarbon oil which can be obtained from ExxonMobil® (Marcol® 52).

In yet another embodiment the inactivated *Lawsonia intracellularis* antigen comprises killed whole cell *Lawsonia intracellularis* bacteria, preferably at a load such that the vaccine comprises *Lawsonia intracellularis* antigen corresponding to 1x10⁷ *Lawsonia intracellularis* bacteria per dose. A higher antigen load, which is not excluded in this embodiment, may positively influence the level of protection and duration of immunity. With regard to the PRRSv load of the vaccine, in an embodiment the vaccine comprises 4.0 log10 (4 units of a 10 log) TCID₅₀ of the attenuated PRRS virus per dose. A higher antigen load, which is not excluded in this embodiment, may positively influence the level of protection and duration of immunity.

The invention will be further explained using the following example and figures. Example 1 describes a study with a PRRS virus and Lawsonia combination vaccine
Figure 1 shows body temperatures post challenge
Figure 2 shows average daily weight gain (ADWG) post challenge
Figure 3 shows PPRS virus serology post challenge
Figure 4 shows Lawsonia serology post challenge
Figure 5 Shows PRRS virus viremia post challenge

Example 2 describes a second study with a PRRS virus and Lawsonia combination vaccine.

### EXAMPLE 1

### STUDY DESIGN

The progeny of five sows (23 piglets) were used for this trial. When the piglets were approx. two weeks old they were vaccinated as follows:
- A first group (Group 1, 8 animals) were vaccinated (IM) with freeze-dried Porcilis® PRRS vaccine (available from MSD Animal Health, Boxmeer, The Netherlands) dissolved using Diluvac Forte® (MSD Animal Health). A single dose contained a calculated amount of 4 log10 TCID₅₀ of virus in 2 ml (the injected dose) and was given into the right side of the neck.
- A second group (Group 2, 9 animals) were vaccinated with the same PRRS vaccine dissolved in a ready-to-use *Lawsonia intracellularis* vaccine (see WO 2009/127684, example 2 for the antigens: killed whole cells, in this experiment formulated in an oil-in-water emulsion, comprising 12.5 % v/v (= volume oil over total volume of the vaccine) of the mineral oil Marcol® 52 (ExxonMobil), 0.75% w/v vitamin E acetate and 0.80% Polysorbate 80 (Tween 80; Sigma Aldrich) and water for injection). The vaccines were mixed at room temperature and left for up to 30 minutes before administration. A single dose contained a calculated amount of 4 log₁₀ TCID₅₀ of virus and 2x10⁷ bacterial cells in 2 ml (the injected dose) and was given into the right side of the neck.
- Piglets in a third group (Group 3, 6 animals) were not vaccinated but served as non-vaccinated challenge controls. Challenge infection was done 4 weeks after vaccination using virulent PRRS challenge virus by the intranasal route.

After vaccination and challenge all piglets were observed daily for clinical signs. Body temperatures were measured one day before and on the day of infection and daily thereafter for ten days. Body weights were determined on the day before challenge, ten days after challenge and one day before the end of the experiment (day 27 post challenge). Blood samples were collected at the time of challenge and three, five, seven, ten, 14, 21 and 28 days later.

### EXPERIMENTAL PROCEDURES

### Veterinary examination

On the day before vaccination, the piglets were examined for general health.

### Observation for clinical signs

Starting on the day of arrival all pigs were observed daily for clinical signs. Starting on the day of challenge individual recording of possible systemic reactions such as loss of appetite, reluctance to move, tendency to lie down, listless or drowsy, shivering, bristling and possibly oedema, especially around the eyes, vomiting and diarrhoea or tachypnea and abdominal breathing was done.

### Measurements of body temperature

Body temperatures of all animals were taken one day before and on the day of challenge and daily thereafter for 10 days (same time of day).

### Measurements of bodyweight

Bodyweights of the piglets were measured on the day before challenge, 10 and 27 days after challenge.

### Sampling of blood

A blood sample from all sows was taken on the day before vaccination to confirm the negative status. At the time of challenge and three, five, seven, ten, 14, 21 and 28 days later blood samples were taken from the piglets. Serum samples were examined for the possible presence of PRRS virus and the possible presence of PRRSV-specific antibodies and *Lawsonia*-specific antibodies.

### RESULTS

### Clinical signs

No clinical reactions after vaccination or challenge were observed during the course of the experiment. Unless the fact that mineral oil is a very harsh adjuvant, in particular for pigs, which may lead to severe local site reactions, it appears that the use of this adjuvant is safe for the new combination vaccine. For any milder adjuvants, safety is thus understood to be no issue at all.

### Body temperature

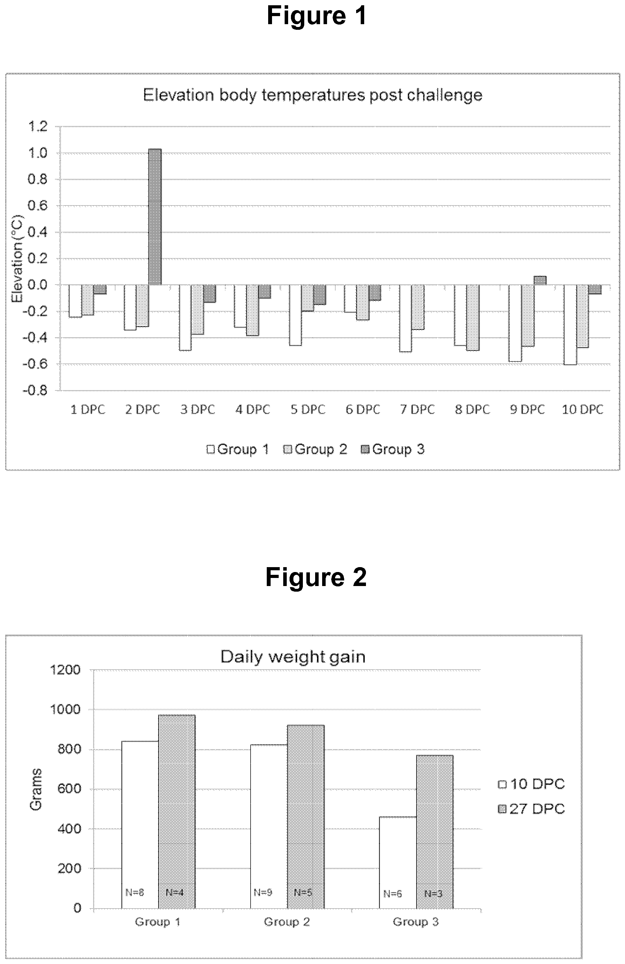

The average pre-challenge body temperature measured one day before and on the day of challenge was calculated for each group. Figure 1 shows the elevation of the average body temperatures until day 10 pc (post challenge) in relation to the pre-challenge average body temperature. On day 2 pc only in the group of the non-vaccinated control piglets the average body temperature was elevated by 1° C. At all other post-challenge time points no elevation was measured neither in the group of the vaccinated nor in the control group piglets.

### Bodyweight and weight gain

At the start of the experiment the piglets were assigned to the groups at random, irrespective of starting weight and gender. As can be seen in Figure 2 the trend is that the combined vaccine of Group 2 provides the same ADWG (average daily weight gain) as the single PRRSV vaccine, higher than the controls.

### PRRSV serology

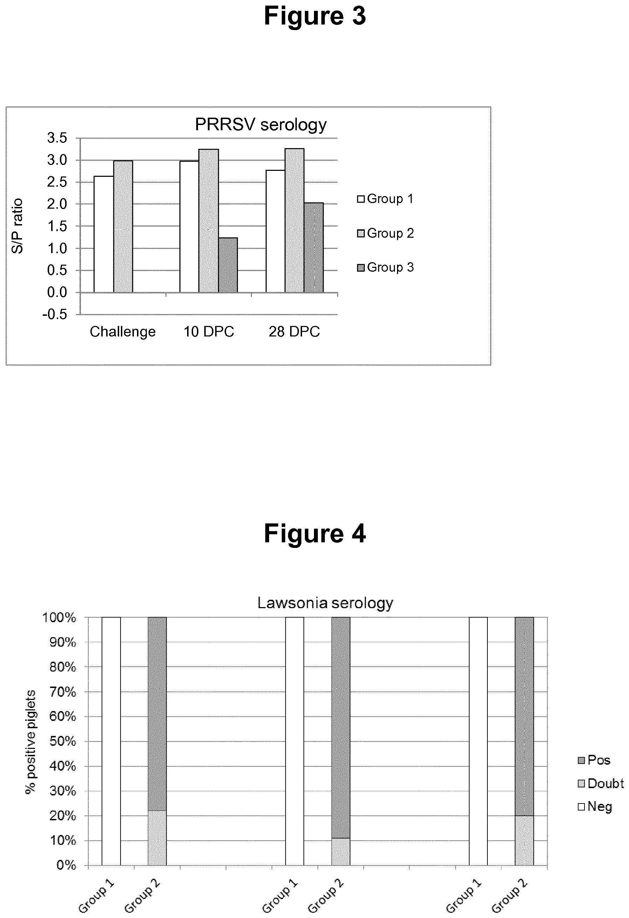

Only pigs that received a PRRS vaccine are positive at challenge. As can be seen in Figure 3 (results are indicated as "sample to Positive or S/P" ratio, using the IDEXX PRRS X3 ELISA), the anti PRRS titer appears to be not negatively influenced by the Lawsonia antigens. On the contrary, there seems to be an unexpected positive effect for the anti-PRRS titer since at each point in time the PRRS titer is higher for the animals that received the combination vaccine. This implies that the Lawsonia antigens for some yet not understood reason, enhance the immunological reaction against the live PRRS virus.

### Lawsonia intracellularis serology

Figure 4 gives the results for the Lawsonia serology. As can be seen, the animals in Group 1 remained negative throughout the experiment. Of the animals in Group 2, 80-90% gave good seroconversion, comparable to the conversion that corresponds to animals protected against virulent Lawsonia challenge.

### PRRSV viremia after challenge

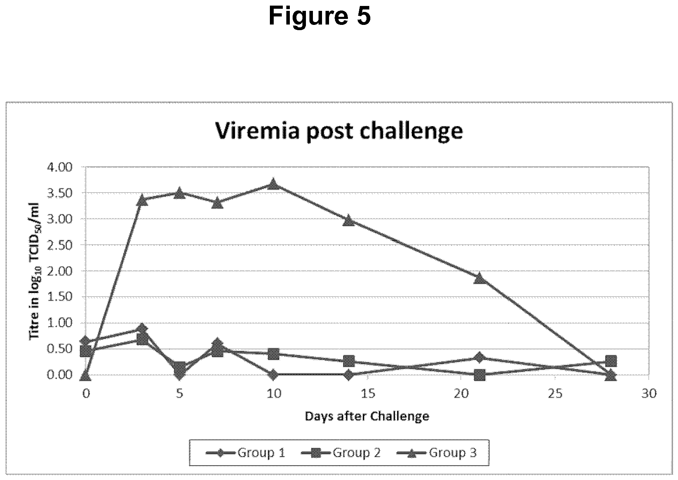

As is commonly known, viremia is a major read-out for protection against PRRS virus infection, and the resulting clinical disease. The results are given in Figure 5. Both groups 1 and 2 have a very high and comparable reduction in viremia.

### EXAMPLE 2

### STUDY DESIGN

This study was designed to confirm that the present combination vaccine is independent of the type of adjuvant and type of live PRRS strain. For this, alternative adjuvants Diluvac Forte (obtainable from MDS Animal Health, Boxmeer, The Netherlands) and Carbopol (obtainable as Carbopol 974P from Lubrizol, Cleveland, Ohio, USA) were used. The alternative PRRSv strain is a type 2 strain (instead of the type 1 strain used in Example 1) as present in the commercially available vaccine Prime Pac PRRS (obtainable from Merck Animal Health, Millsboro, Delaware, USA).

The progeny of several sows were used to allocate 15 piglets to 3 treatment groups of five piglets. At the age of approximately 1 week, piglets of groups 1 and 2 were vaccinated with freeze-dried inactivated Lawsonia vaccine (the same antigens as used in Example 1, but now freeze-dried and thus in combination with a freeze-dry stabilizer) reconstituted in Diluvac Forte (DF) or Carbopol (0.8% w/v) as listed in table 1 below. Vaccines were administered intramuscular (IM) in the left side of the neck. At the age of approximately 5 weeks, the piglets were (re)vaccinated with two different live PRRSV strains and the Lawsonia antigens, combined in one vaccine by reconstitution of the antigens in either DF or Carbopol (0.8% w/v). PRRSV Type 2 vaccine Prime Pac PRRS was dissolved to contain an amount of 10⁴⁵ TCID₅₀ of virus in 2 ml and PRRSV Type 1 vaccine Porcilis PRRS was dissolved to contain an amount of 10^{4.0} TCID₅₀ of virus in 2 ml. For the second vaccination, vaccines were administered intramuscular (IM) in the right side of the neck. Piglets of group 3 were not vaccinated and served as non-vaccinated controls.

**Table 1 vaccination scheme**

| Group | First vaccination | Second vaccination |
|---|---|---|
| 1 | Lawsonia, freeze-dried reconstituted in DF, 2ml | Combination vaccine of Lawsonia, freeze-dried and type 2 live PRRSv in DF, 2 ml |
| 2 | Lawsonia, freeze-dried reconstituted in Carbopol, 2 ml | Combination vaccine of Lawsonia, freeze-dried and type 1 live PRRSv in carbopol, 2 ml |
| 3 | - | - |

After the first vaccination piglets were observed daily for clinical signs. Blood samples from all piglets were taken before the first vaccination, before the second vaccination, and at two, four and six weeks after the second vaccination. Serum samples were checked for antibodies against PRRS virus and *Lawsonia intracellularis.*

### RESULTS

No clinical reactions were observed during the course of the experiment. All animals were negative for antibodies against *Lawsonia* and PRRS on the day of the second vaccination. At six weeks after the second vaccination all animals, except for the controls, were positive for *Lawsonia* and PRRSv specific antibodies. This proves that the combination vaccine was safe and led to active immunization against *Lawsonia intracellularis* and PRRS virus.

## Claims

1. A vaccine comprising in combination an adjuvant, a live attenuated PRRS virus and an inactivated *Lawsonia intracellularis* antigen, wherein the inactivated *Lawsonia intracellularis* antigen comprises killed whole cell *Lawsonia intracellularis* bacteria.

2. A vaccine comprising in combination an adjuvant, a live attenuated PRRS virus and an inactivated *Lawsonia intracellularis* antigen for use in the protection of a swine against an infection with PRRS virus and *Lawsonia intracellularis* bacteria, wherein the inactivated *Lawsonia intracellularis* antigen comprises killed whole cell *Lawsonia intracellularis* bacteria.

3. A vaccine comprising in combination an adjuvant, a live attenuated PRRS virus and an inactivated *Lawsonia intracellularis* antigen for use in the protection of a swine against a clinical disease that results from an infection with PRRS virus and *Lawsonia intracellularis* bacteria, wherein the inactivated *Lawsonia intracellularis* antigen comprises killed whole cell *Lawsonia intracellularis* bacteria.

4. A vaccine according to any of the claims 2 and 3, **characterised in that** the vaccine is for use in the protection of a swine against an infection with PRRS virus and *Lawsonia intracellularis* bacteria after a single shot administration.

5. A vaccine according to claim 1, **characterised in that** the adjuvant comprises a mineral oil.

6. A vaccine for use according to any of the claims 2 to 4, **characterised in that** the adjuvant comprises a mineral oil.

7. A vaccine according to claim 1, **characterised in that** the vaccine comprises *Lawsonia intracellularis* antigen corresponding to 1x10⁷ *Lawsonia intracellularis* bacteria per dose.

8. A vaccine for use according to any of the claims 2 to 4, **characterised in that** the vaccine comprises *Lawsonia intracellularis* antigen corresponding to 1x10⁷ *Lawsonia intracellularis* bacteria per dose.

9. A vaccine according to claim 1, **characterised in that** the vaccine comprises 4.0 log10 TCID₅₀ of the attenuated PRRS virus per dose.

10. A vaccine for use according to any of the claims 2 to 4, **characterised in that** the vaccine comprises 4.0 log10 TCID₅₀ of the attenuated PRRS virus per dose.

## Patentansprüche

1. Impfstoff, umfassend in Kombination ein Adjuvans, ein lebendes abgeschwächtes PRRS-Virus und ein inaktiviertes *Lawsonia intracellularis-*Antigen*,* wobei das inaktivierte *Lawsonia intracellularis-*Antigen abgetötete ganze Zellen von *Lawsonia intracellularis-*Bakterien umfasst.

2. Impfstoff, umfassend in Kombination ein Adjuvans, ein lebendes abgeschwächtes PRRS-Virus und ein inaktiviertes *Lawsonia intracellularis-*Antigen zur Verwendung beim Schutz eines Schweins vor einer Infektion mit PRRS-Virus und *Lawsonia intracellularis-*Bakterien, wobei das inaktivierte *Lawsonia intracellularis*-Antigen abgetötete ganze Zellen von *Lawsonia intracellularis-*Bakterien umfasst.

3. Impfstoff, umfassend in Kombination ein Adjuvans, ein lebendes abgeschwächtes PRRS-Virus und ein inaktiviertes *Lawsonia intracellularis-*Antigen zur Verwendung beim Schutz eines Schweins vor einer klinischen Erkrankung, die von einer Infektion mit PRRS-Virus und *Lawsonia intracellularis-*Bakterien herrührt, wobei das inaktivierte *Lawsonia intracellularis-*Antigen abgetötete ganze Zellen von *Lawsonia intracellularis-*Bakterien umfasst.

4. Impfstoff nach einem der Ansprüche 2 und 3, **dadurch gekennzeichnet, dass** der Impfstoff zur Verwendung beim Schutz eines Schweins vor einer Infektion mit PRRS-Virus und *Lawsonia intracellularis-*Bakterien nach einer Einzelschuss-Verabreichung dient.

5. Impfstoff nach Anspruch 1, **dadurch gekennzeichnet, dass** das Adjuvans ein Mineralöl umfasst.

6. Impfstoff zur Verwendung nach einem der Ansprüche 2 bis 4, **dadurch gekennzeichnet, dass** das Adjuvans ein Mineralöl umfasst.

7. Impfstoff nach Anspruch 1, **dadurch gekennzeichnet, dass** der Impfstoff *Lawsonia intracellularis-*Antigen umfasst, das 1 x 10⁷ *Lawsonia intracellularis* Bakterien pro Dosis entspricht.

8. Impfstoff zur Verwendung nach einem der Ansprüche 2 bis 4, **dadurch gekennzeichnet, dass** der Impfstoff *Lawsonia intracellularis*-Antigen umfasst, das 1 x 10⁷ *Lawsonia intracellularis* Bakterien pro Dosis entspricht.

9. Impfstoff nach Anspruch 1, **dadurch gekennzeichnet, dass** der Impfstoff 4,0 log10 TCID₅₀ des abgeschwächten PRRS-Virus pro Dosis umfasst.

10. Impfstoff zur Verwendung nach einem der Ansprüche 2 bis 4, **dadurch gekennzeichnet, dass** der Impfstoff 4,0 log10 TCID₅₀ des abgeschwächten PRRS-Virus pro Dosis umfasst.

## Revendications

1. Vaccin comprenant, en combinaison, un adjuvant, un virus PRRS atténué vivant et un antigène de *Lawsonia intracellularis* inactivé, où l'antigène de *Lawsonia intracellularis* inactivé comprend des bactéries *Lawsonia intracellularis* à cellules entières tuées.

2. Vaccin comprenant, en combinaison, un adjuvant, un virus PRRS atténué vivant et un antigène de *Lawsonia intracellularis* inactivé, pour une utilisation dans la protection d'un porc contre une infection par le virus PRRS et des bactéries *Lawsonia intracellularis,* où l'antigène de *Lawsonia intracellularis* inactivé comprend des bactéries *Lawsonia intracellularis* à cellules entières tuées.

3. Vaccin comprenant, en combinaison, un adjuvant, un virus PRRS atténué vivant et un antigène de *Lawsonia intracellularis* inactivé, pour une utilisation dans la protection d'un porc contre une maladie clinique résultant d'une infection par le virus PRRS et des bactéries *Lawsonia intracellularis,* où l'antigène de *Lawsonia intracellularis* inactivé comprend des bactéries *Lawsonia intracellularis* à cellules entières tuées.

4. Vaccin selon l'une quelconque des revendications 2 et 3, **caractérisé en ce que** le vaccin est destiné à une utilisation dans la protection d'un porc contre une infection par le virus PRRS et des bactéries *Lawsonia intracellularis,* après une administration en une seule injection.

5. Vaccin selon la revendication 1, **caractérisé en ce que** l'adjuvant comprend une huile minérale.

6. Vaccin pour une utilisation selon l'une quelconque des revendications 2 à 4, **caractérisé en ce que** l'adjuvant comprend une huile minérale.

7. Vaccin selon la revendication 1, **caractérisé en ce que** le vaccin comprend un antigène de *Lawsonia intracellularis* correspondant à 1×10⁷ bactéries *Lawsonia intracellularis* par dose.

8. Vaccin pour une utilisation selon l'une quelconque des revendications 2 à 4, **caractérisé en ce que** le vaccin comprend un antigène de *Lawsonia intracellularis* correspondant à 1×10⁷ bactéries *Lawsonia intracellularis* par dose.

9. Vaccin selon la revendication 1, **caractérisé en ce que** le vaccin comprend 4,0 log10 TCID₅₀ du virus PRRS atténué par dose.

10. Vaccin pour une utilisation selon l'une quelconque des revendications 2 à 4, **caractérisé en ce que** le vaccin comprend 4,0 log10 TCID₅₀ du virus PRRS atténué par dose.
